# EUROPEAN PATENT APPLICATION

(11) **EP 4 799 563 A1**
(43) Date of publication of application: **02.09.2026**
(21) Application number: 25161132.3
(22) Date of filing: 28.02.2025
(51) Int. Cl.: A61B 5/145, A61B 5/1477, A61B 5/1486, A61B 5/00, C12Q 1/00, G01N 27/327

(54) **LACTATE SENSOR**

(71) Applicant: Idro, 2100 Antwerpen-Deurne (BE); Fundacion Universitaria San Antonio, 30107 Guadalupe - Murcia (ES)
(72) Inventor: CUARTERO BOTIA, Maria, 11428 Stockholm (SE); CRESPO PARAVANO, Gaston Adrian, 11346 Stockholm (SE); XUAN, Xing, 30830 La Ñora (ES); CHEN, Chen, 11417 Stockholm (SE); ALONSO SALINAS, Ramiro, 30007 Murcia (ES)
(74) Representative: Calysta NV

(57) **Abstract**

A wearable device comprising a sweat biosensor (1) for lactate, comprising: a sweat collection inlet (2) arranged in the device for collecting sweat when the device is sworn by a user, a microfluidic channel for conveying the collected sweat from the inlet to the sweat biosensor (1), a lactate biosensor (3), which operates by means of amperometry comprising a working electrode (4) comprising an immobilized lactate oxidase and a redox molecule film under a constant negative potential to reduce the said redox molecule, wherein the measuring electrode (4) of the said lactate biosensor (3) comprises a diffusion-limiting membrane comprising PVC and dioctyl sebacate in a weight ratio 1:3 to 2:1 and wherein the processing unit is hermetically sealed (6).

## Description

### Technical Field

The present invention relates to a wearable device for measuring lactate concentration is sweat.

### Prior art

It is known in the prior art to assess the lactate levels to reflect the anaerobic metabolic activity. During exercise, especially in high-intensity activities, the body utilizes the anaerobic pathway to generate energy. In this process, when the energy demand exceeds the capacity of aerobic metabolism, pyruvate (one of the products of glycolysis) is converted into lactate through the action of the enzyme lactate dehydrogenase, producing ATP in the process. This lactate is not simply a waste byproduct; rather, it acts as an alternative fuel and an indicator of the balance between production and clearance in the muscle, which influences the onset of fatigue and the training response.

Lactate measurement is key to determining the aerobic and anaerobic thresholds, fundamental parameters for planning training and improving athletic performance. Traditionally, it has been done using a blood sample, but lactate monitoring in sweat offers a non-invasive and continuous alternative. This allows real-time tracking of the athlete's metabolic response, enabling immediate adjustments in training intensity, preventing overtraining, and optimizing individualized planning.

Similarly, tumors may be hypoxic, which will, again, produce higher lactate levels, at least locally.

The measurement of sweat lactate levels has been proposed as a more practical alternative: this could be an indicator for both sport performance and physiology. However, according to Xuan et al., Analysis and Sensing, 2022, doi.org/10.1002/anse.202200047, among the ten studies identified, four have found an inverse relationship between sweat lactate levels, one study was unconclusive and five suggested a positive relationship between lactate and exercise.

To overcome these discrepancies, Xuan et al developed a wearable sweat lactate sensor, where sweat is continuously measured and the usefulness of measuring sweat as an indicator of sport performance has been suggested. In practice Xuan et al have developed an epidermal patch comprising a lactate biosensor, a sweat inlet connected to a microfluidic channel, in which the electrodes needed for the amperometry readout (working, reference, and counter) were placed, followed by the outlet. With such a design, once the inlet is full, the sweat flows through the channel until it reaches the outlet. The sweat is continuously replenished in the inlet, and thus in the channel due to active perspiration. The working electrode is a lactate biosensor based on measuring the peroxide (H₂O₂) formed as a subproduct in the reaction of lactate with the lactate oxidase enzyme.

A stable linear range for lactate measurement in sweat has been reported between 1 and 20 mM, with a correlation coefficient of 0.813 to blood lactate levels. However, this correlation weakens as exercise intensity increases, leading to spikes in lactate levels in both sweat and blood: a significant portion of valuable exercise data might be overlooked, particularly at higher intensities where lactate surpasses the 20 mM threshold.

This limitation highlights a significant drawback of the device. Despite its potential, it fails to capture the full range of lactate concentrations, especially during high-intensity exercise, where levels can reach up to 40-45 mM. Consequently, crucial physiological information could be missed, reducing the device's effectiveness in accurately monitoring exercise performance.

### Brief summary of the invention

A first aspect of the present invention relates to a wearable device 1 to be fixed on the skin of a user for providing lactate measurement in real-time, the said device 1 comprising:
- a sweat collection inlet 2 arranged in the device 1 for collecting sweat when the device 1 is sworn by the said user,
- a microfluidic channel 9 for conveying the collected sweat from the inlet 2 to a lactate biosensor 3, which operates by means of amperometry, said lactate biosensor 3 comprising a working electrode 4 comprising an immobilized lactate oxidase, chitosan and a redox molecule film,
- a processing unit 5 adapted for receiving and processing data provided by the lactate biosensor 3,
wherein the working electrode 4 of the said lactate biosensor 3 comprises a diffusion-limiting membrane comprising PVC and dioctyl sebacate in a weight ratio from 1:3 to 2:1 and wherein the processing unit 5 is hermetically sealed 6 and wherein the working electrode 4 measures the current generated upon oxidation of the said redox molecule.

Preferably, this device is further comprising a reference electrode 7 comprising a polyvinyl butyral layer, and being preferably an Ag/AgCl electrode.

Preferably, the diffusion membrane of the working electrode 4 consists essentially of PVC, dioctyl sebacate, and
tetradodecylammonium tetrakis(4-chlorophenyl)borate (ETH500), or tridodecylmethylammonium chloride (TDMACL).

Preferably, all the electrodes including, the counter, the reference 7 and the working electrode 4 are made of graphene, advantageously produced by a CO2 laser over a polyimide film, possibly applied with a power between 40% and 60%. Preferably, in this process, the laser power is fixed between 10 and 60 W, preferably between 15 and 40 W, more preferably between 20 and 30W. Preferably, in this process, the laser wavelength is comprised between about 8000 and 15000 nm, preferably between about 9000 and 12000 nm, more preferably is of (about) 10,600 nm. Advantageously, the laser speed is comprised between 10 and 100 mm/s, preferably between 20 and 75 mm/s, more preferably between 40 and 50 mm/s.

Advantageously, this device is to be applied to the skin in a sealed fashion, preferably wherein the processing unit 5 is sealed with a silicon and/or a neoprene barrier 6.

Preferably, the lactate oxidase is immobilized in a chitosan network, advantageously obtained upon addition of chitosan solution (1%).

Preferably, the redox molecule is Prussian Blue (Prussian White), tetrathiafulvalene or grafted-polymerized MgO-templated carbon, preferably Prussian Blue (Prussian White), preferably wherein the Prussian Blue (Prussian White) layer has been applied by drop-casting.

Preferably, in this device, the working electrode 4 is placed at less than 2 cm from the sweat collection inlet 2.

Preferably, this device comprises no pH and/or temperature probe(s).

Preferably one, two, or the three electrodes including, the counter, the reference 7 and the working electrode 4 are made of graphene, advantageously produced by a CO2 laser over a polyimide film, possibly applied with a power between 40% and 60%. Preferably, in this use, the electrode(s) are obtained using a laser power between 10 and 60 W, preferably between 15 and 40 W, more preferably between 20 and 30W. Preferably, in this use, the electrode(s) are obtained using a laser wavelength is comprised between about 8000 and 15000 nm, preferably between about 9000 and 12000 nm, more preferably is of (about) 10,600 nm. Advantageously, the laser speed to generate the electrode(s) is comprised between 10 and 100 mm/s, preferably between 20 and 75 mm/s, more preferably between 40 and 50 mm/s.

A related aspect of the present invention is the use of one or several graphene electrode(s) for measuring lactate concentration in sweat.

A related aspect of the present invention is a process for the large-scale production of this device 1, comprising the following steps:
- deposition of an aqueous composition comprising the redox molecule on the working electrode 4 and of evaporating the water,
- deposition of an aqueous composition having a viscosity of less than 1000 cP (25°C) comprising lactate oxidase and chitosan on the measuring electrode coated with the said redox molecule and of evaporating the water, and
- deposition of the diffusion-limiting membrane on the lactate-oxidase coated working electrode 4 being in a composition comprising THF/Cyclohexanone, preferably in a volume ratio comprised between 25:75 and 75:25 (THF:cyclohexanone), and of evaporating the said THF/Cyclohexane.

Preferably, in this process, the diffusion-limiting membrane consists essentially of, PVC, dioctyl sebacate and tetradodecylammonium tetrakis(4-chlorophenyl)borate (ETH500).

Preferably this process further comprises the step of applying a polyvinyl butyral layer on a reference electrode 8, the said polyvinyl butyral to be applied being in a dispersion in an organic phase, the said dispersion having a viscosity of less than 500 cP, and of evaporating the organic phase after application of the said composition on the said reference electrode 8.

Preferably, the pressure exerted on the plunger introducing the solutions into the device is controlled and is within 200 and 400 kPa.

Preferably, the dispenser shutter opening time and/or the dispenser shutter closing time ranges from 0.15 milliseconds to 1 millisecond.

Preferably, in this process, the electrode(s) are graphene electrode(s) obtained as described here above using a CO2 laser.

Preferably, the redox molecule is Prussian Blue and/or the deposition method of the redox molecule is by drop casting.

### Brief description of the Drawings

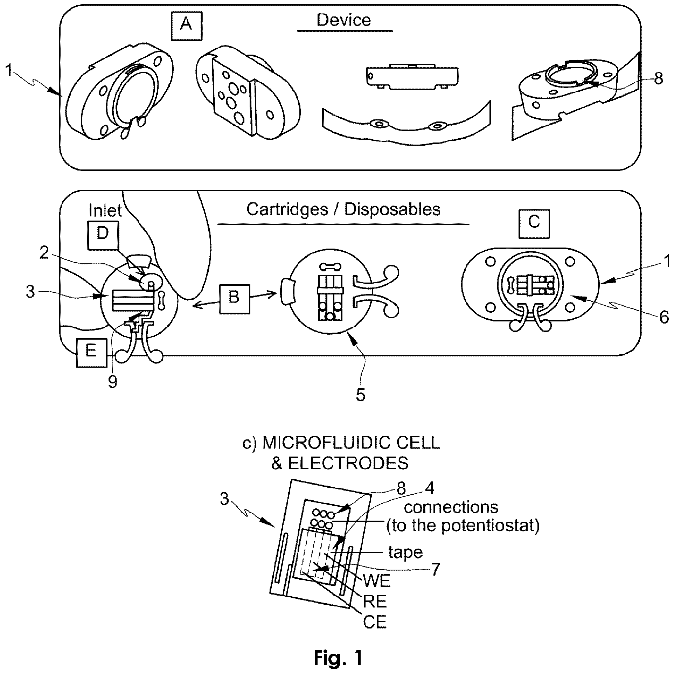

Figure 1 is a schematic view of the wearable device according to the invention
Figure 2 shows the current/concentration relationship of different formulations of the barrier membrane.

In the drawings, the same reference numbers have been allocated to the same or analogue element.

### Detailed description of the invention

The inventors have developed a unique expertise in wearable devices for lactate sensors in sweat. In their development work, they have found a diffusion-limiting membrane allowing (i) a broad linear response rate, (ii) a reasonably good sensitivity, (ii) a reasonable limit of detection and (iiii) an acceptable response time.

They have also found a process to apply the different layers on the working (measuring) electrode at an industrial scale and how to arrange the different elements so as to avoid leakage while ensuring a correct flux of sweat from the user's body through the working (measuring) electrode.

A first object of the present invention is a wearable device 1 for measurement in real-time of lactate concentration in sweat, the said device 1 comprising:
a sweat collection inlet 2 arranged in the device for collecting sweat when the device 1 is sworn by a user,
a microfluidic channel 9 for conveying the collected sweat from the inlet 2 to a lactate biosensor 3,
the said lactate biosensor 3, which operates by means of amperometry, comprising a working electrode 4 comprising an immobilized lactate oxidase within a chitosan network and a redox molecule film (or a film comprising a redox molecule),
a processing unit 5 adapted for receiving and processing data provided by the sweat lactate sensor,
wherein the working (measuring) electrode 4 of the said lactate biosensor 3 comprises a diffusion-limiting membrane and wherein the said lactate biosensor 3 provides lactate measurement in real-time, wherein the diffusion-limiting membrane of the working (measuring) electrode comprises PVC and dioctyl sebacate in a weight ratio comprised between 1:4 to 2:1, preferably between 1:3 and 1:1 and wherein the processing unit is hermetically sealed 6.

This wearable device is to be applied on the skin.

Preferably, the working (measuring) electrode 4 is placed at less than 2 cm from the inlet 2.

This ensures a rapid and unbiased measurement of the lactate concentration in sweat.

Preferably, a current is applied to reduce the redox molecule and/or to ensure a minimal amount of the redox molecule in a reduced state.

This allows to reflect the H2O2 production generated by the immobilized lactate oxidase when contacted to sweat lactate.

Preferably the device of the present invention does not comprise pH probe and/or pH sensor.

The inventors have found that these additional sensors, although appearing to provide interesting complementary information, are difficult to combine with the lactate sensor (working, measuring electrode 4) since the collected sweat does not reach all the sensors at the same time.

Preferably, the diffusion membrane of the working (measuring) electrode 4 consists essentially of PVC, dioctyl sebacate (DOS) and either tetradodecylammonium tetrakis(4-chlorophenyl)borate (ETH500) or tridodecylmethylammonium chloride (TDMACI), preferably the ETH500 or the TDMACI being incorporated in a weight percent between 2 and 10% (weight ETH500 or TDMACl: weight ETH500/TDMACl+weight PVC+weight DOS), preferably between 2.2 and 8, more preferably between 2.4 and 7 %; ETH500 is preferred over TDMACl.

The inventors have found that the combined use of PVC and dioctyl sebacate at the right relative ratios allows a good optimum between the above-listed parameters.

Preferably, the diffusion-limiting membrane is 10 µm thick and/or has a weight comprised between 200 and 1000 µg.

Preferably, this device 1 further comprises a reference electrode 7 comprising a polyvinyl butyral layer. A PVB membrane saturated with chloride in the reference electrode ensures stable potential and accurate measurements in amperometric lactate detection in sweat. It enhances durability and protects against contaminants, ensuring reliable long-term performance.

Preferably, the lactate oxidase is immobilized on, or in, a porous network or (porous) resin. The inventors have identified that chitosan is advantageous to act as a porous network to anchor the enzyme.

This allows to gently immobilize the enzyme as well as the diffusion of lactate.

Preferably, the redox molecule is Prussian Blue (iron hexacyanoferrate; CAS 14038-43-8), tetrathiafulvalene, or grafted-polymerized MgO-templated carbon. The most preferred redox molecule is Prussian Blue; the inventors have found that the molecule is easily reduced by an electric source into Prussian White, then oxidized by reacting with the co-product of the lactate oxidase, hydrogen peroxide.

Advantageously, this device is applied to the skin in a sealed fashion, preferably the processor is isolated from the sweat through a tight junction 6, preferably with an additional silicon barrier and/or with an additional neoprene barrier.

Preferably, all the electrical connections are protected by insulating material such as rubber.

A related aspect of the present invention is a process for the large-scale production of the above device 1 comprising the following steps:
- deposition of an aqueous composition comprising the redox molecule on the working (measuring) electrode 4 and of evaporating the water,
- deposition of an aqueous composition having a viscosity of less than 1000 cP (25°C), preferably of less than 500 cP, more preferably of less than 100 cP, still more preferably of less than 50 cP comprising lactate oxidase and chitosan on the working (measuring) electrode 4 and of evaporating the water, and
- deposition of the diffusion-limiting membrane in a composition comprising THF/Cyclohexanone on the working (measuring) electrode 4, preferably in a volume ratio comprised between 25:75 and 75:25 (THF:cyclohexanone), and of evaporating the said THF/Cyclohexane.

The inventors have found that the viscosity parameter is very important because higher viscosities risk to block the dispersing head for the solution/suspension, especially the suspension for the membrane. Similarly, the inventors have found that the THF risks to evaporates too quickly, but that mixtures with cyclohexanone overcomes this issue.

Preferably, in such process, the diffusion-limiting membrane consists essentially of, PVC, dioctyl sebacate and tetradodecylammonium tetrakis(4-chlorophenyl)borate (ETH500).

This process preferably further comprises the step of applying a polyvinyl butyral layer on a reference electrode 7, the said polyvinyl butyral to be applied being in a dispersion in an organic phase, the said dispersion having a viscosity of less than 500 cP, preferably of less than 300 cP, still more preferably of less than 200 cP and of evaporating the organic phase after application of the said composition on the said reference electrode.

Such a process is advantageously performed at room temperature (e.g. between 15°C and 30°C, preferably at about 20°C) and at atmospheric pressure.

Other characteristics and advantages of the present invention will be derived from the non-limitative following description, and by making reference to the drawings and the examples.

It should be understood that the present invention is not limited to the described embodiments and that variations can be applied without going outside of the scope of the claims.

### Examples

The inventors have started from their own publication of Xuan et al.

### Example 1 - Components of the barrier membrane.

The working (measuring) electrode to be incorporated in the wearable device is firstly recovered with a layer where a redox molecule is embedded; here, Prussian Blue (Prussian white, to be oxidized into Prussian Blue), then with a layer comprising immobilized lactate oxidase and chitosan, and finally with a diffusion-limiting membrane.

The Prussian Blue (Prussian white) layer is applied in a water suspension, so as the lactate oxidase suspension (after the first layer has been dried). The diffusion-limiting membrane is then applied in an organic solvent, such as DMF, THF and cyclohexanone. In this Example, the inventors used THF as the organic solvent.

For the diffusion-limiting membrane, the inventors have selected PVC membranes, to be formulated with either ETH500 or TDMACL (tridodecylmethylammonium chloride), and with DOS, according to the Table 1 here below.

**Table 1: Composition of the membranes assayed ^{a}**

| **Membrane** | **Weight (mg) of the components** | | | |
|---|---|---|---|---|
| | **PVC/mg** | **DOS/mg** | **ETH500/mg** | **TDMACl/mg** |
| M1 | 33 | 66 | | |
| M2 | 33 | 66 | 3 | |
| M3 | 33 | 66 | 6 | |
| M4 | 33 | 66 | 9 | |
| M5 | 33 | 66 | | 3 |
| M6 | 33 | 66 | | 6 |
| M7 | 33 | 66 | | 9 |
| M8 | 50 | 50 | 6 | |
| M9 | 66 | 33 | 6 | |

| | | | | |
|---|---|---|---|---|
| ^{a}All components are prepared and dissolved in 1 mL of THF. | | | | |

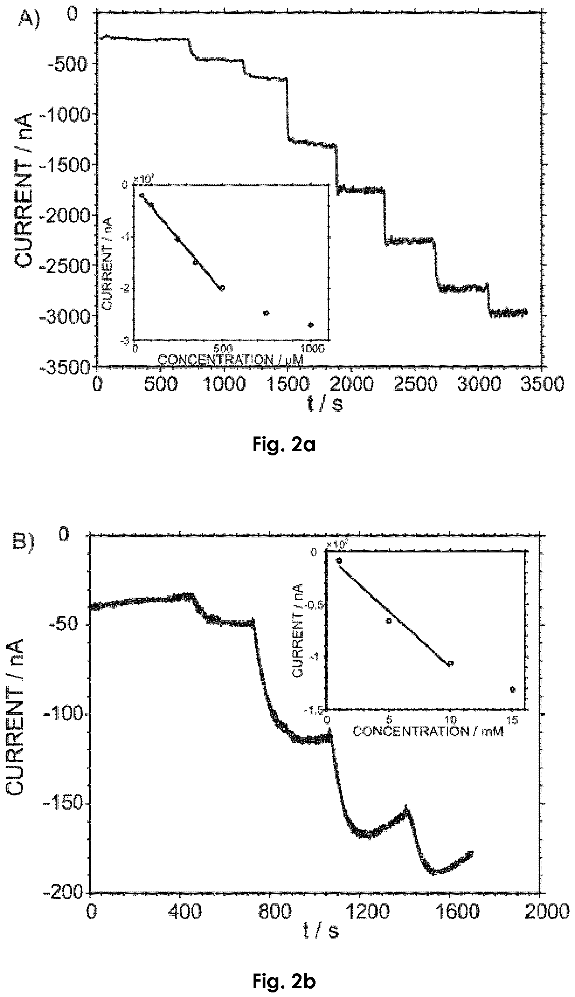

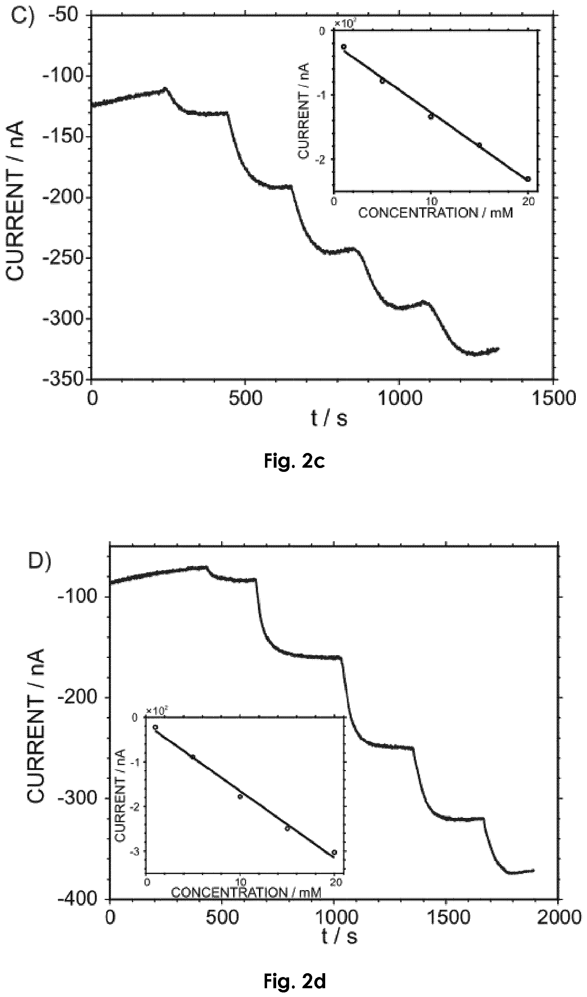

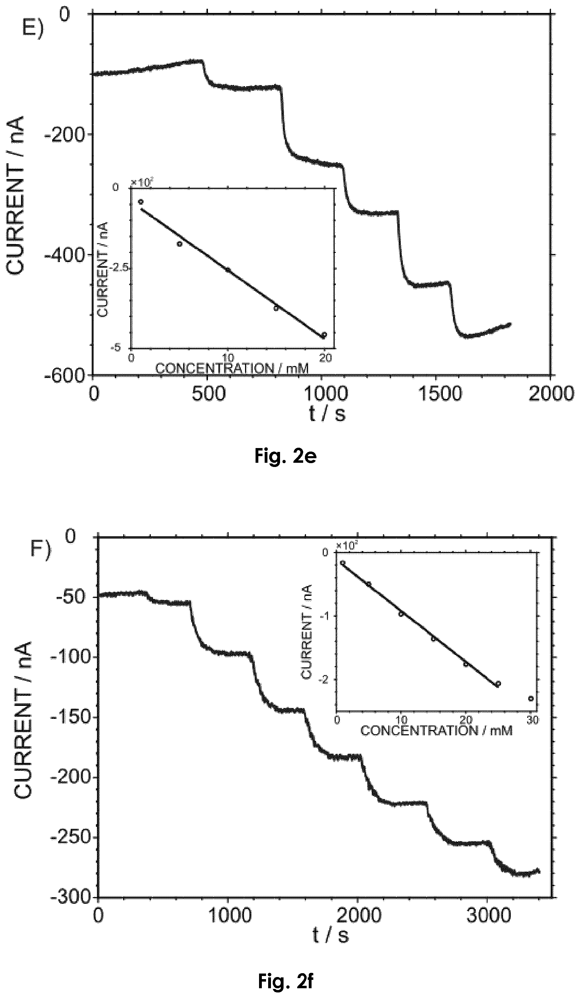

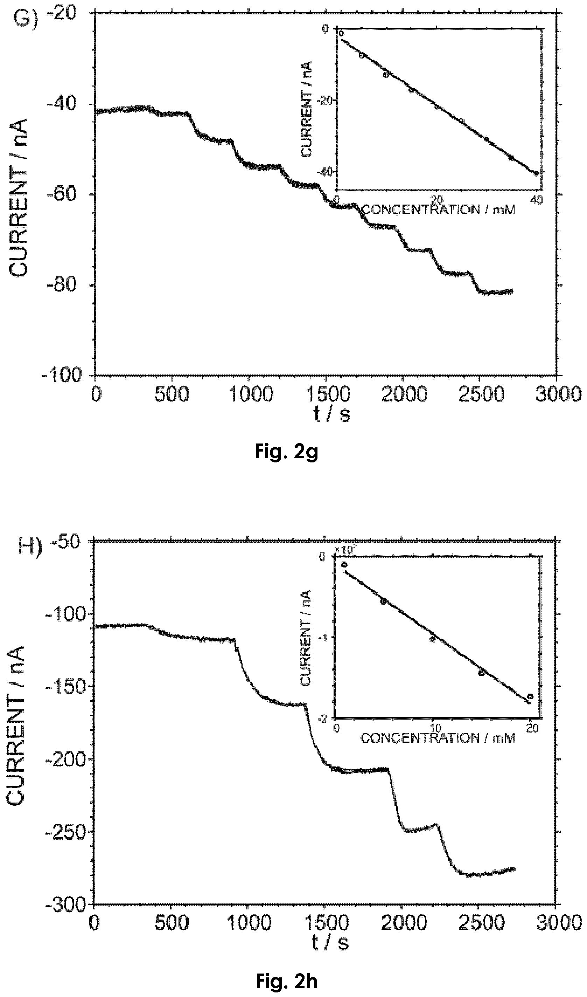

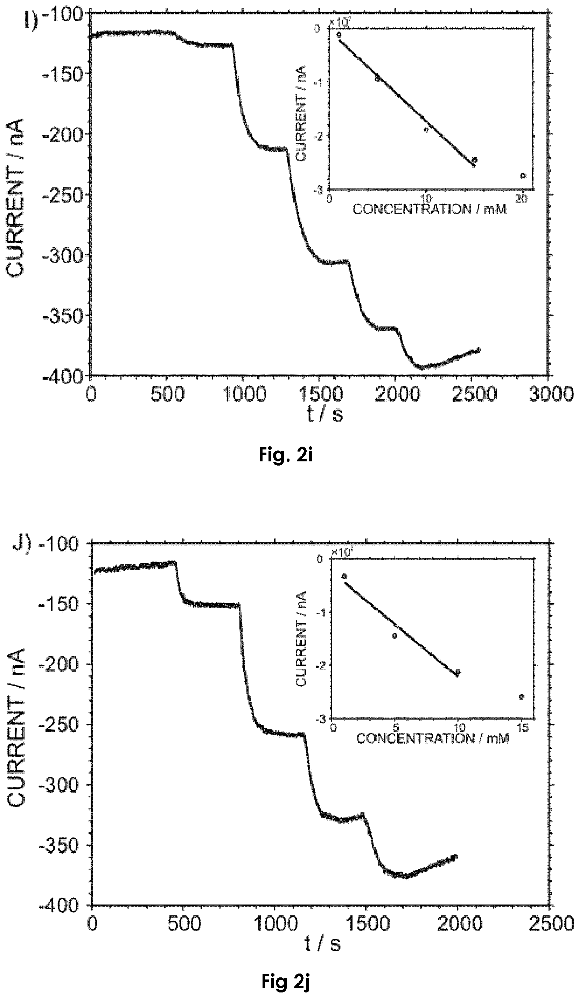

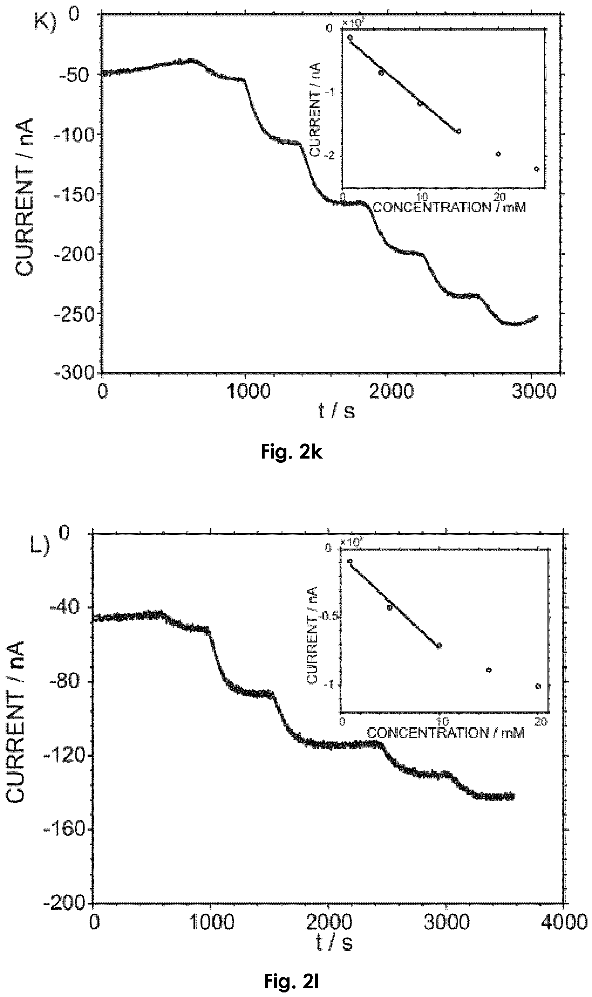

Then the inventors have tested the wearable devices with the different diffusion-limiting membranes (see Figure 2); the µl values indicate the amount of the composition used to form the diffusion-limiting membrane.

More into details, dynamic responses and corresponding calibration curves (insets) have been carried out in phosphate buffer solution 0.1 M at increasing lactate concentration using A) no diffusion limiting membrane, B) M1 membrane, C) M2 membrane, D) M3 membrane, E) M4 membrane, F) M3 membrane, prepared with 6µL membrane cocktail, G) M3 membrane, prepared with 7.5 µL membrane cocktail, H) M5 membrane, I) M6 membrane, J) M7 membrane, K) M8 membrane, and L) M9 membrane. 3 µL of membrane cocktail was used for drop-casting preparation of each membrane, except for the conditions F and L (6 µl) and G (7.5 µl).

The polymeric membranes act as diffusion-limiting elements in the lactate biosensor, expanding the sensing range to the millimolar level (>10 mM) compared to the case without membrane, Figure 2a (<0.5 mM). However, the dynamic responses of the biosensor are significantly influenced by the composition of the membrane. Particularly noteworthy was the effect of introducing a lipophilic salt (Figure 2c - l), which enhanced the linear range as compared (>15 mM) to membranes without the lipophilic salt (Figure 2b, <10 mM).

The main results are summarized at the Table 2

| Membrane | Linear range / mM | **a t / s^{a} 95%** | **Sensitivity** / **nA mM⁻¹** | **LOD / mM** |
|---|---|---|---|---|
| Without membrane | 0.05 - 0.5 | 28.33 ± 5.75 | 3.87 ± 0.16 (nA µM⁻¹) | 0.033 |
| M1 | 1 - 10 | 197.50 ± 7.53 | 11.49 ± 0.46 | 0.51 |
| M2 | 1 - 20 | 123.33 ± 7.67 | 10.25 ± 0.42 | 0.24 |
| M3 (3 µL) | 1 - 20 | 106.67 ± 7.67 | 14.58 ± 0.51 | 0.29 |
| M3 (6 µL) | 1 - 25 | 176.67 ± 36.10 | 8.08 ± 0.30 | 0.66 |
| M3 (7.5 µL) | 1 - 40 | 187.33 ± 11.33 | 1.18 ± 0.27 | 1.51 |
| M4 | 1 - 20 | 55.50 ± 9.67 | 22.80 ± 1.24 | 0.31 |
| M5 | 1 - 20 | 187.73 ± 9.67 | 9.45 ± 1.19 | 0.35 |
| M6 | 1-15 | 125.67 ± 17.50 | 16.97 ± 0.68 | 0.39 |
| M7 | 1-10 | 91.33 ± 23.33 | 21.60 ± 1.44 | 0.23 |
| M8 | 1-15 | 223.33 ± 20.72 | 9.31 ± 0.79 | 0.33 |
| M9 | 1-10 | 210.25 ± 15.45 | 7.41 ± 0.52 | 0.47 |

| | | | | |
|---|---|---|---|---|
| ^{a} The response time (t_{95%}) was calculated with the highest concentration within the linear range. | | | | |

As shown in Table 2 or Figure 2, the dynamic range can be expanded, at the expense of the detection limit or of the sensitivity. However, the presence of the diffusion-limiting membrane also prolonged the response time.

In a subsequent run of experiments, the inventors have developed a membrane with PVC 33; DOS 66 and ETH550 2.5 with good results.

The inventors have also adapted the working electrode 4 and have found that a working electrode 4 comprising graphene provides better results over a working electrode being a screen printed carbon electrode.

### Example 2; Process for scaling-up

The inventors have found that THF, which is used in Example 1 for manual dispensing, evaporates extremely quickly and risks blocking the dispensing head of the composition for the diffusion-limiting membrane, making the deposition process irreproducible; on the other hand, the inventors have found that the mixture of THF with cyclohexanone overcomes this issue.

Regarding droplet size and the amount of solution dispensed, the trials revealed five adjustable parameters that influence the size and shape of the dispensed solution: viscosity of the solution; pressure exerted on the plunger introducing the solution into the device; dispenser shutter opening time (measured in milliseconds); dispenser shutter closing time (measured in milliseconds); pressure exerted by the device during dispensing (between 60% and 70% of the pressure allowed in the device; EV2 with a PICOPulse nozzle from Nordson EFD), allowing for configurations such as circular or linear dispensing. The smallest droplet size tested was 0.08 microliters, while the largest was on the order of milliliters. The current dispensing configuration comfortably fits within this range.

On the other hand, the inventors have compared electrochemical deposition of Prussian Blue and drop-casting of Prussian Blue; the latter allows an expanded linear range, reduced response time and detection limit, while maintaining sensitivity.

## Claims

1. A wearable device (1) to be fixed on the skin of a user for providing lactate measurement in real-time, the said device (1) comprising:
- a sweat collection inlet (2) arranged in the device (1) for collecting sweat when the device (1) is sworn by the said user,
- a microfluidic channel (9) for conveying the collected sweat from the inlet (2) to a lactate biosensor (3), which operates by means of amperometry, said lactate biosensor (3) comprising a working electrode (4) comprising an immobilized lactate oxidase, chitosan and a redox molecule film,
- a processing unit (5) adapted for receiving and processing data provided by the lactate biosensor (3),
wherein the working electrode (4) of the said lactate biosensor (3) comprises a diffusion-limiting membrane comprising PVC and dioctyl sebacate in a weight ratio from 1:3 to 2:1 and wherein the processing unit (5) is hermetically sealed (6) and wherein the working electrode (4) measures the current generated upon oxidation of the said redox molecule.

2. The device of claim 1 further comprising a reference electrode (7) comprising a polyvinyl butyral layer, and being preferably an Ag/AgCl electrode.

3. The device of claim 1 or 2, wherein the diffusion membrane of the working electrode (4) consists essentially of PVC, dioctyl sebacate, and
tetradodecylammonium tetrakis(4-chlorophenyl) borate (ETH500), or tridodecylmethylammonium chloride (TDMACL).

4. The device according to any one of the preceding claims, wherein the working electrode (4) comprises a graphene layer and more preferably is made of graphene and/or is a graphene electrode.

5. The device according to any one of the preceding claims being applied to the skin in a sealed fashion, preferably wherein the processing unit (5) is sealed with a silicon and/or a neoprene barrier (6).

6. The device according to any one of the preceding claims, wherein the lactate oxidase is immobilized on a chitosan network.

7. The device according to any one of the preceding claims, wherein the redox molecule is Prussian Blue, tetrathiafulvalene or grafted-polymerized MgO-templated carbon, preferably Prussian Blue, preferably wherein the Prussian Blue layer has been applied by drop-casting.

8. The device according to any one of the preceding claims wherein the working electrode (4) is placed at less than 2 cm from the sweat collection inlet (2).

9. The device according to any one of the preceding claims comprising no pH and/or temperature probe(s).

10. A process for the large-scale production of the device (1) according to any one of the preceding claims comprising the following steps:
- deposition of an aqueous composition comprising the redox molecule on the working electrode (4) and of evaporating the water,
- deposition of an aqueous composition having a viscosity of less than 1000 cP (25°C) comprising lactate oxidase and chitosan on the measuring electrode coated with the said redox molecule and of evaporating the water, and
- deposition of the diffusion-limiting membrane on the lactate-oxidase coated working electrode (4) being in a composition comprising THF/Cyclohexanone, preferably in a volume ratio comprised between 25:75 and 75:25 (THF:cyclohexanone), and of evaporating the said THF/Cyclohexane.

11. The process of claim 10, wherein the diffusion-limiting membrane consists essentially of PVC, dioctyl sebacate and tetradodecylammonium tetrakis(4-chlorophenyl)borate (ETH500), and/or wherein the redox molecule is Prussian Blue and/or wherein the deposition method of the redox molecule is by drop casting.

12. The process of claim 10 or 11 further comprising the step of applying a polyvinyl butyral layer on a reference electrode (8), the said polyvinyl butyral to be applied being in a dispersion in an organic phase, the said dispersion having a viscosity of less than 500 cP, and of evaporating the organic phase after application of the said composition on the said reference electrode (8).

13. The process according to any one of the preceding claims 10 to 12, wherein the pressure exerted on the plunger introducing the solution into the device is controlled and is within 200 and 400 kPa.

14. The process according to any one of the preceding claims 10 to 13, wherein the dispenser shutter opening time and/or the dispenser shutter closing time ranges from 0.15 milliseconds to 1 milliseconds.

15. The process according to any one of the preceding claims 11 to 14, wherein one or all the electrode(s) is (are) are made of graphene produced by a CO2 laser over a polyimide film, preferably, at a laser power fixed between 10 and 60 W, and/or preferably at a speed comprised between 10 and 100 mm/s.
